# EUROPÄISCHE PATENTSCHRIFT

(11) **EP 1 509 189 B1**
(45) Veröffentlichungstag und Bekanntmachung des Hinweises auf die Patenterteilung: **08.09.2010**
(21) Anmeldenummer: 03730104.1
(22) Anmeldetag: 23.05.2003
(51) Int. Cl.: A61K 6/033

(54) **INDUZIERTE REMINERALISATION VON HUMANEM ZAHNSCHMELZ**
INDUCED REMINERALISATION OF HUMAN TOOTH ENAMEL
REMINERALISATION INDUITE D'EMAIL HUMAIN

(30) Priorität: 24.05.2002 DE 10223157
(43) Veröffentlichungstag der Anmeldung: 02.03.2005
(73) Patentinhaber: Max-Planck-Gesellschaft zur Förderung der Wissenschaften e.V., 80539 München (DE)
(72) Erfinder: BUSCH, Susanne, 61267 Neu-Anspach (DE); KNIEP, Rüdiger, 40764 Langenfeld (DE)
(74) Vertreter: Dey, Michael
(86) Internationale Anmeldenummer: PCT/EP2003/005430
(87) Internationale Veröffentlichungsnummer: WO 2003/099234

(56) Entgegenhaltungen:
- WO-A-94/10969
- WO-A-98/10736
- DE-A- 2 131 666
- DE-A- 2 350 548
- DE-A- 3 303 937

## Beschreibung

Die vorliegende Anmeldung betrifft die induzierte Remineralisation von humanem Zahnschmelz und insbesondere den Aufbau von Apatit auf Zahnmaterial.

Zähne sind Verbundstoffe aus Apatit und Proteinen. Es handelt sich bei ihnen um sehr harte Biomaterialien auf der Basis von Calcium und Phosphat. Der Zahnschmelz, die äußere Schicht der Zahnkrone ist der härteste Teil des Zahns und enthält keine lebenden Zellen. Zahnschmelz besteht aus anorganischen Kristallen, welche typische hoch orientierte Anordnungen aufweisen. Zahnschmelz ist ein Gewebe, das, sobald es einmal gebildet ist, lebenslang nahezu unverändert bleibt, da die Zellen, welche beim Aufbau der Zähne beteiligt sind, absterben, sobald die Zahnbildung abgeschlossen ist. Ein fertiger Zahnschmelz besteht aus etwa 95 Gew.-% Apatit, etwa 3 Gew.-% Proteinen und Lipiden und etwa 2 Gew.-% Wasser.

Um Schädigungen von Zähnen, insbesondere durch Karies zu vermeiden oder zu reparieren, wurde seit langem versucht, remineralisierende Systeme einzusetzen. Dabei wurde zunächst versucht, durch Aufbringen von Calciumphosphatverbindungen die Beschaffenheit der Zähne zu verbessern. Solche Einkomponentensysteme, bei denen versucht wird, bereits vorgefertigtes Zahnmaterial, beispielsweise Apatit, Hydroxyapatit oder andere Calciumphosphatverbindungen auf die Zähne aufzubringen, sind unter anderem in EP 0 666 730 B1 oder WO 01/95863 beschrieben. Das Problem solcher Systeme besteht darin, dass das Behandeln von Zahnmaterial mit Calciumphosphatverbindungen nicht zu einem Aufwachsen von strukturell dem Zahnmaterial ähnlichem Apatit führt, sondern vielmehr zu einer bloßen Anlagerung von Apatitkristallen auf dem Zahnmaterial, wobei die Apatitkristalle eine vom Zahnmaterial völlig unterschiedliche Morphologie aufweisen. Somit wird keine Festigung des Zahnschmelzes oder dauerhafte Füllung von Läsionen bewirkt, da die angelagerten Apatitkristalle keine ausreichende Ähnlichkeit und Haftung zum natürlichen Zahnmaterial aufweisen.

Weiterhin wurde versucht mit Zweikomponentensystemen eine Remineralisierung von Zähnen zu erhalten, wobei die Systeme üblicherweise eine Calciumphase sowie eine Phosphatphase umfassen. Zweikomponentensysteme sind beispielsweise in WO 98/10736 und DE 33 03 937 A1 beschrieben. Nachteilig bei den dort beschriebenen Vorgehensweisen ist, dass die in WO98/10736 beschriebene Methode Calcium- und Phosphatlösungen vor der Anwendung vereinigt, sodass sich eine metastabile Lösung bildet, aus der auf dem Zahn Apatit auskristallisieren soll. Die Methode erlaubt keine lokalisierte Behandlung am Zahn, da das Reagenz als Mundspülung oder Gel eingesetzt wird, das mit der Zahnbürste einmassiert wird. Weiterhin wird die Kompositnatur des nativen Schmelzes nicht berücksichtigt, da im System keine organische Kommponente enthalten ist. Die Bildung zahnschmelzähnlicher Kristallite ist demnach unwahrscheinlich. DE 33 03 937 beschreibt ein Verfahren, bei dem Calcium- und Phosphationen getrennt nacheinander auf den Zahn aufgebracht werden, indem dieser in eine Kappe getaucht wird, welche die entsprechenden Ionen in einer Gelatinematrix enthält. Bei einer empfohlenen Einwirkungszeit von nur zwei Minuten ist nicht zu erwarten, dass sich wirklich größere Mengen Apatit auf der Zahnoberfläche bilden können. Es ist nicht mit Bildmaterial belegt, dass die neugebildete Apatitschicht schmelzähnliche Strukturen aufweist.

In weiteren Arbeiten (S. Busch et al., Eur. J. Inorg. Chem. (1999), 1643-1653; S. Busch et al., Chem. Mater. 13 (2001), 3260-3271; S. Busch, Zahnärztliche Mitteilungen 91, Nr. 10 (2001), 34-38; R. Kniep et al., Angew. Chem. 108, Nr. 22 (1996), 2787-2791) wurde die biomimetische Morphogenese von Fluorapatit-Gelatinkompositen untersucht. Dabei wurde biomimetisches Wachstum und Selbstorganisation von Fluorapatit-Aggregaten durch Diffusion in denaturierten Collagen-Matrizes beobachtet. Die Grundlagen der Fluorapatitbildung in Gelatine-Gelen wurde dabei mittels Doppeldiffusionsversuchen von Calcium- und Phosphatlösungen in einem U-Rohr untersucht. Diese Arbeiten beschreiben die Bildung von Fluorapatitkügelchen innerhalb des verwendeten Gels.

Eine Aufgabe der vorliegenden Anmeldung war es, ein Verfahren bereitzustellen, mit dem Defekte an Zahnmaterial durch Remineralisation ausgebessert werden können. Eine weitere Aufgabe war es, Zahnmaterial mit einer schützenden Apatitschicht zu bedecken.

Diese Aufgabe wird erfindungsgemäß gelöst durch ein Verfahren zum Aufwachsen von Apatit auf Zahnmaterial, umfassend die Schritte:
(i) Auftragen eines ersten Gels, welches Gelatine sowie Phosphationen umfasst,
(ii) Auftragen eines zweiten Gels, wobei mit diesem zweiten Gel die erste Gelschicht abgedeckt wird und
(iii) Auftragen eines Calciumionen enthaltenden Mediums,
wobei ein Aufbau von Apatit an der Oberfläche des Zahnmaterials bewirkt wird.

Erfindungsgemäß ist es überraschenderweise möglich, ein wirkliches Aufwachsen von Zahnschmelz-ähnlichem Material zu erzielen. Ein wesentlicher Vorteil besteht darin, dass eine hohe Ordnung kleiner Apatitnadeln erhalten wird, die strukturell eine große Ähnlichkeit zu nativem Zahnschmelz aufweisen. Bei entsprechender Substratorientierung ist praktisch kein Unterschied zwischen aufgewachsenem Apatit und ursprünglichem Zahnmaterial zu erkennen.

Weitere Vorteile der Erfindung sind, dass von einem echten Aufwachsen der Fluorapatitkristallite auf dem Zahnsubstrat ausgegangen werden kann, wie die REM-Bilder belegen. Die Vickers-Härte dieser neuen Schicht entspricht dem natürlichen Schmelz. Die Durchführung der einzelnen Schritte ist so einfach, dass die Remineralisation von Zahnschmelz im Prinzip vom Patienten selbst durchgeführt werden kann. Das Gel kann lokal auf die geschädigten Stellen aufgetragen werden und verfestigt sich dort. Da das erwärmte Gel sehr schnell abkühlt, sind kaum Wartezeiten zwischen den einzelnen Schritten nötig.

Da die Erweichungstemperatur des Gels etwas über der normalen Körpertemperatur liegt (38 bis 42 °C), wird ein Schmelzen des Gels während der Einwirkungsdauer verhindert. Damit kann eine unkontrollierte Mineralisation vermieden werden.

Durch Zugabe von Fluoridionen zum phosphathaltigen Gel kann die Resistenz der Schicht gegenüber Säuren erhöht werden.

Erfindungsgemäß ist es möglich durch induzierte Remineralisation Zahnschmelzdefekte zu regenerieren. Durch den Einsatz eines zweischichtigen Gels, welches bei Körpertemperatur fest ist und lokal auf die betroffene Stelle am Zahn aufgebracht werden kann sowie durch die Verwendung einer Mundspülung als Calciumionen enthaltenden Medium werden Mineralisationsbedingungen geschaffen, welche die Bildung einer Zahnschmelz-ähnlichen Substanz bewirken, die direkt auf dem Zahn aufwächst. Bei der bisher veröffentlichten Doppeldiffusionsmethode wurde lediglich gezeigt, dass Fluorapatit, der durch Gegenstromdiffusion von Calcium- und Phosphationen in einem Gelatinegel entsteht, kugelige Aggregate bildet, deren organischer Gewichtsanteil dem von reifem, menschlichem Zahnschmelz entspricht. Die Doppeldiffusionsmethode hat aber weder eine Möglichkeit eröffnet, am Menschen die Remineralisation von Zahnschmelz zu ermöglichen noch diese Möglichkeit in irgendeiner Weise impliziert. Der bei der Doppeldiffusionsmethode eingesetzte Versuchsaufbau bewirkt die Bildung von kleinen Kügelchen und erlaubt nicht das Aufwachsen von gleichmäßigen Schichten von Apatitmaterial auf einem Substrat. Dies ist erst durch die erfindungsgemäße Vorgehensweise möglich.

Die Erfindung lässt sich insbesondere beim Menschen anwenden. Dabei können beispielsweise kleinere kariöse Defekte durch induzierte Remineralisation ausgeheilt oder empfindliche Stellen am Zahn mit einer schützenden Apatitschicht bedeckt werden. Die Vorgehensweise zur Behandlung ist dabei bevorzugt wie folgt: Die kariöse Stelle wird zunächst mit einer dünnen Schicht des etwa 50 °C warmen phosphathaltigen Gels bestrichen oder mit einer geeigneten Spritze aufgetragen, die gewärmt werden kann. Das Gel erstarrt sofort auf der Zahnoberfläche und wird nach der gleichen Methode mit dem Schutzgel abgedeckt. 1 bis 3 mal am Tag wird dann mit der Calciumlösung eine etwa 10-minütige Mundspülung durchgeführt. Zwischen den Spülungen wird der Zahn mit einer passenden Kappe abgedeckt, die aus Plastik oder Metall sein kann, sodass der Patient nicht behindert ist und die Remineralisation ungestört stattfinden kann. Wenn viele Zähne betroffen sind, kann auch die ganze Zahnreihe mit einer Schiene geschützt werden, wie sie z.B. gegen Zähneknirschen eingesetzt wird. Alle zwei Tage wird das Gel gewechselt, zu dieser Gelegenheit wird der betroffene Zahn gereinigt und desinfiziert.

Erfindungsgemäß wird zunächst auf das Zahnmaterial ein erstes Gel aufgebracht. Dieses Gel enthält Gelatine sowie Phosphationen und gegebenenfalls weitere Bestandteile. Der Gehalt an Gelatine im ersten Gel beträgt bevorzugt von mindestens 15 Gew.-%, mehr bevorzugt von 25 Gew.-% bis zu 40 Gew.-%, mehr bevorzugt bis zu 30 Gew.-%. Der Gelatine kommt insbesondere eine Funktion bei der Ausbildung der Morphologie des gebildeten Apatits zu. Es wurde überraschenderweise festgestellt, dass bei der Verwendung von Gelatine ein Apatitmaterial an der Oberfläche von Zahnmaterial abgeschieden wird, welches eine große Ähnlichkeit mit nativem Zahnschmelz aufweist. Bei Verwendung anderer organischer Matrizes wurden hingegen andere Morphologien der Apatitkristallisate beobachtet, sodass es nicht zu einem Aufbau von Apatit an der Oberfläche des Zahnmaterials, wie erfindungsgemäß angestrebt, kommt.

Gelatine ist ein Polypeptid, welches insbesondere durch Hydrolyse des in Haut und Knochen von Tieren enthaltenen Collagens gewonnen werden kann. Gelatine weist üblicherweise ein Molekulargewicht von 15.000 bis über 250.000 g/mol auf und kann aus Collagen unter sauren oder alkalischen Bedingungen gewonnen werden. Erfindungsgemäß bevorzugt werden folgende Gelatinen eingesetzt: Sauer hydrolysierte Gelatinesorten (Typ A), z.B. hergestellt aus Schweineschwarte oder Kalbshaut mit hohem Bloom-Wert, z.B. 250 bis 350 Bloom (unter dem Bloomwert versteht man eine Kenngröße, welche die Gelfestigkeit kennzeichnet, im Allgemeinen gilt, je höher der Bloomwert, umso höher der Anteil langkettiger Moleküle in der Gelatine und um so höher die Gelfestigkeit).

Neben Gelatine, welche zur Ausbildung der gewünschten Morphologie des Apatits und den Aufbau an der Oberfläche des Zahnmaterials enthalten ist, umfasst das erste Gel weiterhin Phosphationen. Diese Phosphationen stellen einen Grundbestandteil des aus Calciumphosphat aufgebauten Apatits dar. Die Konzentration der Phosphationen im ersten Gel beträgt bevorzugt mindestens 0,01 mol/l, mehr bevorzugt mindestens 0,05 mol/l und bis zu 0,5 mol/l, mehr bevorzugt bis zu 0,2 mol/l und insbesondere 0,08 mol/l.

Das erste Gel weist bevorzugt eine Erweichungstemperatur auf, die über der normalen Körpertemperatur liegt, sodass das Gel bei Körpertemperatur fest ist. Die Erweichungstemperatur des ersten Gels liegt bevorzugt im Bereich von 38 bis 45 °C, mehr bevorzugt von 38 bis 42 °C. Das erste Gel wird bevorzugt in erwärmter Form, beispielsweise auf 45 bis 55 °C erwärmt aufgetragen. Nach dem Auftragen kühlt das Gel ab und wird fest.

Erfindungsgemäß wird in einem weiteren Schritt ein zweites Gel, ein sogenanntes Schutzgel aufgetragen. Mit diesem zweiten Gel wird insbesondere die erste Gelschicht abgedeckt. Das Schutzgel, welches als Geldeckschicht fungiert, bewirkt überraschenderweise, dass die Mineralisation, also die Bildung von Apatit, überwiegend oder ausschließlich an der Zahnoberfläche und nicht an der Grenzschicht Gel-Flüssigkeit stattfindet. Durch den zweischichtigen Gelaufbau, der beim erfindungsgemäßen Verfahren erzielt wird, kommt es überraschenderweise zu einem Aufbau bzw. Aufwachsen von Apatit auf dem Zahnmaterial und nicht zu einer Kristallisation oder Ausbildung von Apatitkugeln innerhalb des Gels, wie es im Stand der Technik beschrieben wird. Nur durch den zweischichtigen Aufbau ist somit eine praktikable und technisch sinnvolle Remineralisation der Zähne möglich.

Der pH-Wert und die Gelkonzentrationen des zweiten Gels entsprechen typischerweise denen, die hierin für das erste Gel angegeben sind. Auch das zweite Gel weist bevorzugt eine Erweichungstemperatur von 38 bis 45 °C, insbesondere von 38 bis 42 °C auf und wird bevorzugt auf 45 bis 55 °C erwärmt aufgebracht.

In einem dritten Schritt wird schließlich ein Calciumionen enthaltendes Medium aufgebracht. Das Calciumionen enthaltende Medium stellt den weiteren zur Bildung von Apatit benötigten Grundbaustoff, nämlich Calciumionen bereit. Diese Calciumionen diffundieren durch das Schutzgel und die erste Gelschicht bis zur Oberfläche des Zahnmaterials und werden dort als Apatit abgeschieden. Die Konzentration der Calciumionen im Calciumionen enthaltenden Medium beträgt vorzugsweise mindestens 0,01 mol/l, mehr bevorzugt mindestens 0,05 mol/l und bis zu 0,5 mol/l, mehr bevorzugt bis zu 0,2 mol/l und insbesondere 0,13 mol/l.

Es wurde festgestellt, dass erfindungsgemäß überraschenderweise eine gleichmäßige Schicht paralleler oder strahlig gewachsener Apatitkristallite gebildet werden kann. Weiterhin zeigt diese Schicht keinen oder nur einen Submikrometer großen Randspalt zum nativen Zahnmaterial auf. Die Wachstumsrichtung der Apatitkristallite erfolgt unabhängig von der Orientierung der Schmelzprismen senkrecht zum Substrat, sodass bei entsprechender Orientierung der Schmelzprismen die Längsorientierung der künstlich aufgewachsenen Kristalle weitgehend identisch mit den Kristallen in den Prismen verläuft. Die Größenordnung von Schmelzkristallen und aufgewachsenem Fluorapatit ist gleich. Innerhalb der Schichten kann eine dichte und gleichmäßige Packung beobachtet werden. Weiterhin weist die aufgebrachte Apatitschicht eine Vickers-Härte auf, die der vom nativen Zahnschmelz entspricht. Die erfindungsgemäß aufgebrachten Apatitschichten weisen insbesondere eine Vickers-Härte im Bereich von 250 bis 400 HV auf.

Erfindungsgemäß ist es möglich, Apatitschichten in beliebiger Dicke aufzubringen, da die erreichte Schichtdicke von der Häufigkeit des Gelwechsels abhängig ist. Pro Gelwechsel können bisher Schichtdicken von bis zu 1 µm erzielt werden.

In einer bevorzugten Ausführungsform wird als erstes Gel ein Gelatine-Glycerin-Gel eingesetzt. Das Gewichtsverhältnis von Gelatine zu Glycerin beträgt dabei bevorzugt 1:5 bis 5:1, insbesondere 1:2 bis 2:1. Glycerin hat die Wirkung, dass der Erweichungspunkt des Gels über die normale menschliche Körpertemperatur angehoben wird. Die erzielte Gelfestigkeit ist notwendig, um das Zweischichtsystem während der Mineralisation zu erhalten, sodass eine gezielte, kontrollierte Kristallabscheidung ermöglicht wird. In einem flüssigen Gel würde es zu einer spontanen Präzipitation feinkristallinen Materials kommen, welches nicht am Zahn anwächst.

Das erste Gel enthält bevorzugt weiterhin Fluoridionen. Das Fluorid kann beispielsweise als Natriumfluorid oder Ammoniumfluorid zugegeben werden. In dieser Ausführungsform kann Fluor-reicher Apatit oder Fluorapatit auf der Oberfläche des Zahnmaterials aufgewachsen werden. Fluorapatit ist insbesondere säureresistenter als der Carbonat-haltige Hydroxyapatit des natürlichen Zahnschmelzes, wobei die Morphologie der sich bildenden Schichten aus Fluorapatit dennoch eine große Ähnlichkeit mit nativem Zahnschmelz aufweist.

Die Wachstumsgeschwindigkeit des Apatits oder Fluorapatits wird unter anderem durch den pH-Wert des ersten Gels bestimmt. Bevorzugt weist das erste Gel einen pH-Wert von 2,0 bis 6,0, insbesondere von 4,0 bis 6,0, mehr bevorzugt von 5,0 bis 5,5 auf.

Ein wesentliches Merkmal der vorliegenden Erfindung ist die Verwendung eines Schutzgels als zweites Gel. Mit diesem Schutzgel wird die Phosphationen-haltige erste Gelschicht abgedeckt. Durch Verwendung dieser Schutzgelschicht findet überraschenderweise die Apatitbildung ausschließlich an der Oberfläche des Zahnmaterials statt und es kommt nicht zu einer spontanen Auskristallisierung von Apatitkristalliten oder Kompositaggregaten, wie sie bei den im Stand der Technik bekannten Vorgehensweisen beobachtet wird. Im Gegensatz zu den Untersuchungen mit Doppeldiffusionskammern kann somit gezielt eine Beschichtung von Zahnmaterialoberflächen erhalten werden. Das zweite Gel enthält vorzugsweise keine Materialien, die in den Apatit eingebaut werden sollen, ist also insbesondere Phosphationen-, Calciumionen- und/oder Fluorionenfrei. Zur Bildung des zweiten Gels kann ebenfalls Gelatine eingesetzt werden, wobei ein Gelatine-Glycerin-Gel bevorzugt ist. Als zweites Gel kann aber auch ein anderes Gel, z.B. ausgewählt aus Polysacchariden, etwa Agarose oder Carragnan, sowie Carboxymethylcellulose verwendet werden.

Erfindungsgemäß wird schließlich das mit ersten Gel und Schutzgel beschichtete Zahnmaterial mit einem Calcium enthaltenden Medium behandelt. Als Calcium enthaltendes Medium kann beispielsweise eine Calciumionen enthaltende Lösung oder/und ein Calciumionen enthaltendes Gel eingesetzt werden. Das Calciumionen enthaltende Medium wird dabei bevorzugt unter Verwendung eines wasserlöslichen Calciumionen enthaltenden Salzes, beispielsweise aus CaCl₂ hergestellt.

Das Calciumionen enthaltende Medium weist bevorzugt einen pH-Wert von 6 bis 8 auf.

Erfindungsgemäß werden somit die beiden Bestandteile von Apatit, nämlich Phosphationen und Calciumionen jeweils getrennt als eigene Komponente zugeführt, wobei die Calciumphosphatbildung dann erst auf der Zahnmaterialoberfläche stattfindet.

Um ein lokales Übersäuern an der Mineralisationsfront durch die Protonenabgabe bei der Apatitbildung zu verhindern, wird das Phosphatgel bevorzugt mit einem Puffersystem versetzt, bevorzugt einem Essigsäurepuffer oder α-α-α-Tris-(hydroxymethyl)methylamin-Puffer.

Vor der Behandlung mit dem ersten Gel kann das Zahnmaterial vorbehandelt werden, insbesondere entfettet, angeätzt oder/und gespült werden. Beispielsweise kann für eine bessere Wirksamkeit die Zahnoberfläche zunächst mit Ethanol entfettet und mit Phoshorsäure angeätzt und anschließend mit entionisiertem Wasser gespült werden.

Das erfindungsgemäße Verfahren eignet sich insbesondere zur Behandlung von Humanzähnen oder Zahnschmelz. Dabei können kariöse Defekte durch Remineralisation behandelt werden oder aber auch das Zahnmaterial prophylaktisch mit einer schützenden Apatit- oder Fluorapatitschicht bedeckt werden. Die Apatitschichten bilden sich sowohl auf Zahnschmelz als auch auf Dentin als Substrat.

Die Erfindung betrifft weiterhin einen Kit, welcher zur Verwendung im Verfahren gemäß den Ausprüchen 1-10 geeignet ist und
a) ein erstes Gel, welches Gelatine sowie Phosphatioen umfasst,
b) ein zweites Gel, welches frei von Phosphationen, Calciumionen und Fluoridionen ist sowie
c) ein Calciumionen enthaltendes Medium umfasst.

Die bevorzugten Ausgestaltungen des ersten und zweiten Gels sowie des Calciumionen enthaltenden Mediums sind dabei wie oben beschrieben.

Die Erfindung wird durch die beigefügten Figuren und die folgenden Beispiele weiter erläutert.

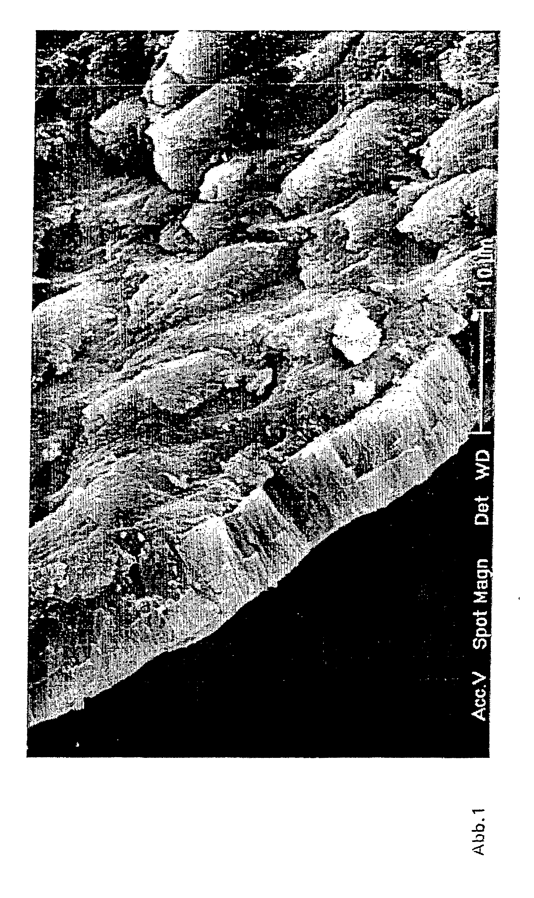

Die Figuren 1 bis 6 zeigen REM-Aufnahmen von auf Zahnmaterial aufgewachsenem Apatit.

### Beispiele

### Beispiel 1

### Schritt 1. Vorbereitung des Zahnmaterials

Ein humaner Zahn (beliebig) wurde von seiner Wurzel getrennt und die Krone in etwa 0,5 mm breite Scheiben gesägt und zwar so, dass die Längsachse der Schmelzprismen schräg zur Schnittrichtung orientiert war. Die Scheiben wurden 30 s lang in einer 30 %-igen Phosphorsäurelösung getaucht, mit entionsiertem Wasser gewaschen und getrocknet.

### Schritt 2. Vorbereitung des Gels

Aus 8,56 g Gelatine, 8,24 g 85 %-iger Glycerinlösung, 7,26 g H₂O, 1,8 ml 2N NaOH, 2,7 ml 2 N HAc, 13,8 mg NaF, 236 mg Na₂HPO₄ wurde bei 80 °C unter Rühren ein homogenes Gel hergestellt, dessen pH-Wert bei 5,0 lag. Ein weiteres Gel wurde aus 8,56 g Gelatine, 8,24 g 85 %-iger Glycerinlösung und 11,76 g H₂O hergestellt. Eine 0,133 molare Calciumlösung wurde aus CaCl₂-Salz hergestellt.

### Schritt 3. Induzierte Mineralisation an der Zahnoberfläche

Die Oberfläche der Zahnscheiben wurde mit etwa 0,5 ml des phoshathaltigen Gels bestrichen. Nach dessen Verfestigung erfolgte eine Bedeckung mit etwa 0,5 ml des zusatzfreien Gels. Die Zahnscheibe wurde in ein einseitig verschlossenes Plastikrohr eingefügt und in einer Calciumlösung bei 37 °C gelagert. Das Gel und die Lösung wurden alle 7 Tage erneuert, insgesamt 16 x. Zur Begutachtung der aufgewachsenen Schicht erfolgte ein Aufbruch der Probe senkrecht zur Schnittfläche, um die Schichtdicke ausmessen zu können. Wie die REM-Aufnahme in Abbildung 1 belegt, bildete sich eine gleichmäßige Schicht elongierter Kristallite mit einer Schichtdicke von 7,2 µm. Das entspricht einer Wachstumsgeschwindigkeit von etwa 450 nm/Woche.

### Beispiel 2

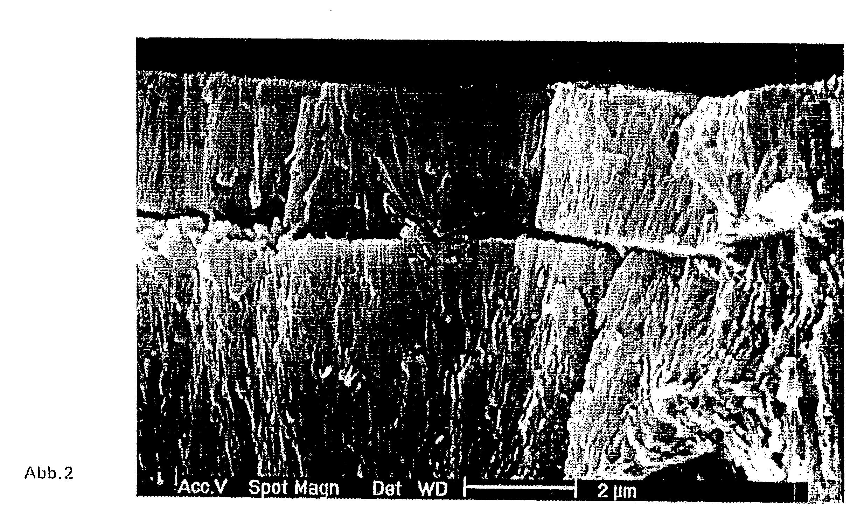

Die Vorgehensweise erfolgt analog Beispiel 1, jedoch lag die Schnittrichtung der Zahnscheiben weitgehend senkrecht zur Längsachse der Schmelzprismen. Zudem wurde die Probe bei 36 °C gelagert und täglich nur 60 min der Calciumlösung ausgesetzt. Das Gel ist alle 2 Tage ausgetauscht worden. Die Probe wurde nach 10 Austauschzyklen untersucht. Die REM-Aufnahme (Abbildung 2) zeigt eine gleichmäßige Schicht parallel angeordneter Kristalle, deren Orientierung und Größenordnung der des nativen Schmelzes entspricht. Ein submikrometer großer Randspalt ist zu erkennen. Die Schichtdicke beträgt 2,7 µm. Das entspricht einer Wachstumsgeschwindigkeit von 135 nm/Tag.

### Beispiel 3

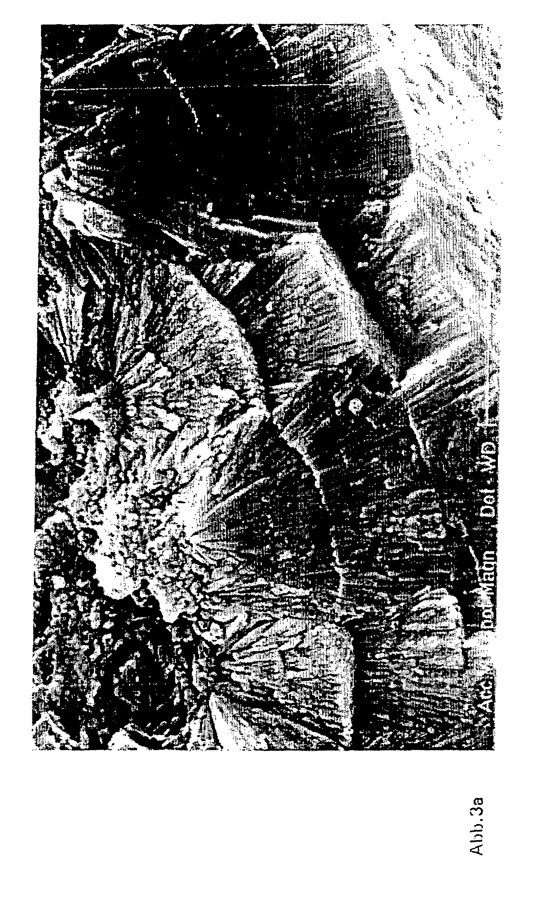

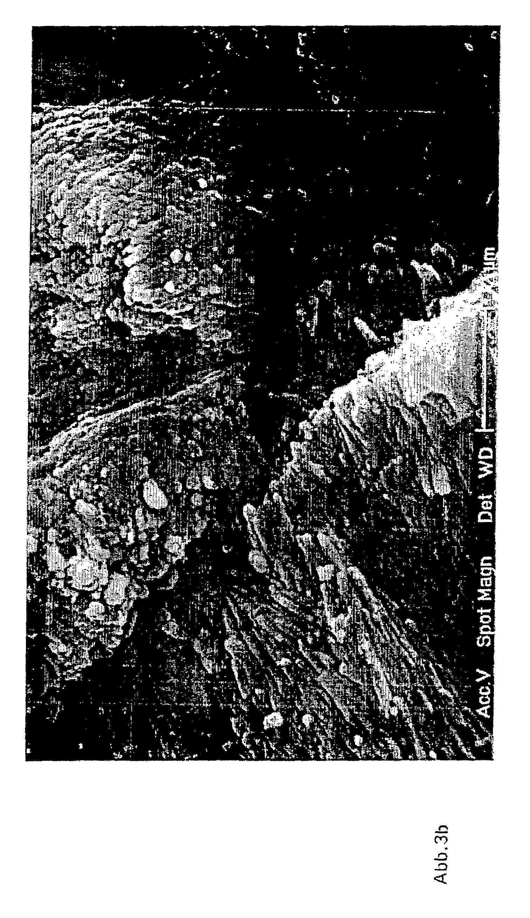

Die Zahnvorbereitung erfolgte analog Beispiel 1, die Schnittrichtung der Scheibe lag jedoch weitgehend parallel zur Längsachse der Schmelzprismen. Die Probe wurde bei 37 °C aufbewahrt, die Austauschzyklen ensprechen dem Beispiel 2, jedoch wurde das Gel insgesamt 36 x erneuert. Die REM-Aufnahme (3a) lässt eine gleichmäßige, 15 µm dicke Schicht erkennen. Dabei ergeben sich ineinander gewachsene, strahlige Strukturen, die eine gute Abdichtung der nativen Zahnoberfläche gewährleisten. Der Randspalt zum Zahn ist minimal, kleinste, morphologische Eigenarten der nativen Oberfläche werden vom aufgewachsenden Material abgebildet (analog einem Abdruck). Größe und Morphologie von Schmelzkristallen und aufgewachsenem Fluorapatit sind gleich (Abbildung 3b). Die Wachstumsgeschwindigkeit liegt bei etwa 210 nm/Tag.

### Beispiel 4

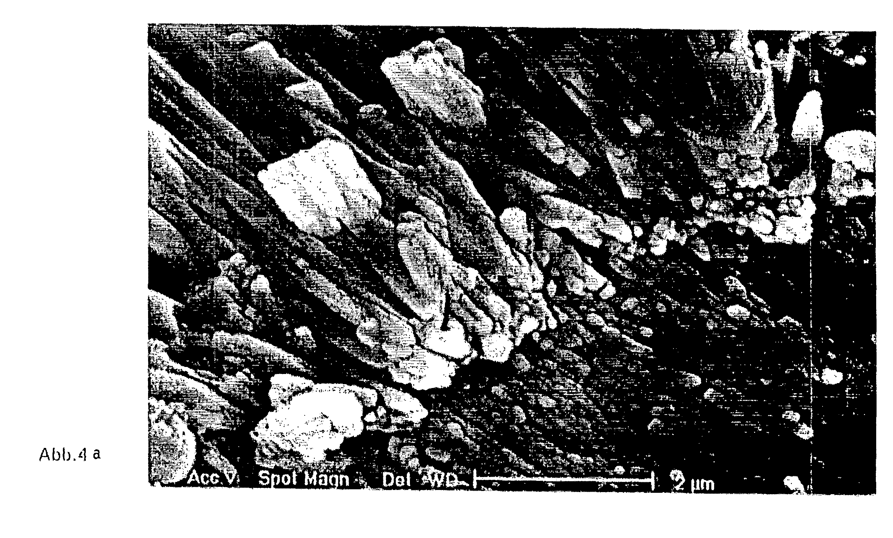

Das Vorgehen entspricht Beispiel 2, aber für das Phosphatgel wurden 755,2 mg eingesetzt und das Gel wurde nur 5 x ausgetauscht. Analog dem Beispiel 2 zeigt die REM-Aufnahme (Abbildung 4a) die übereinstimmende Orientierung von Schmelzkristalliten und aufgewachsenem Fluorapatit. Die Kristallite erscheinen jedoch noch nicht vollständig ausgereift. Die Schichtdicke liegt bei 5 µm, das entspricht einer Wachstumsgeschwindigkeit von 500 nm/Tag.

### Beispiel 5

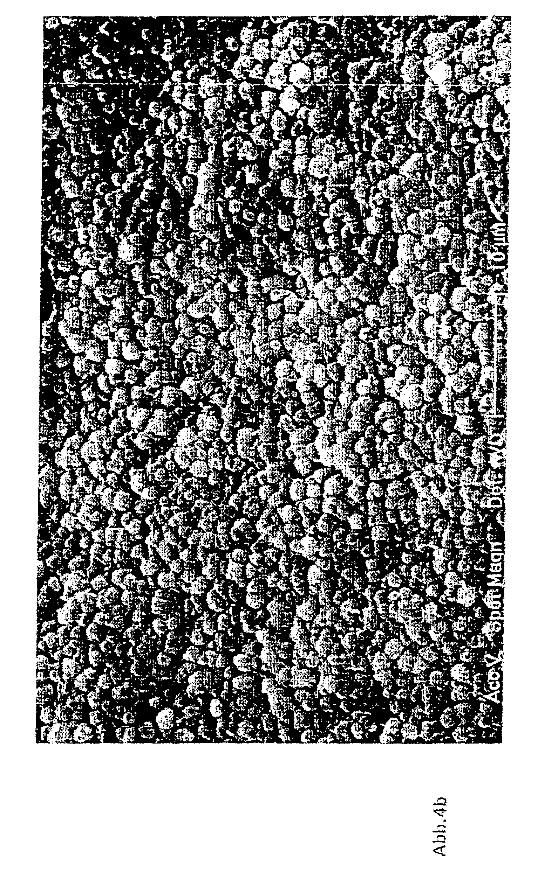

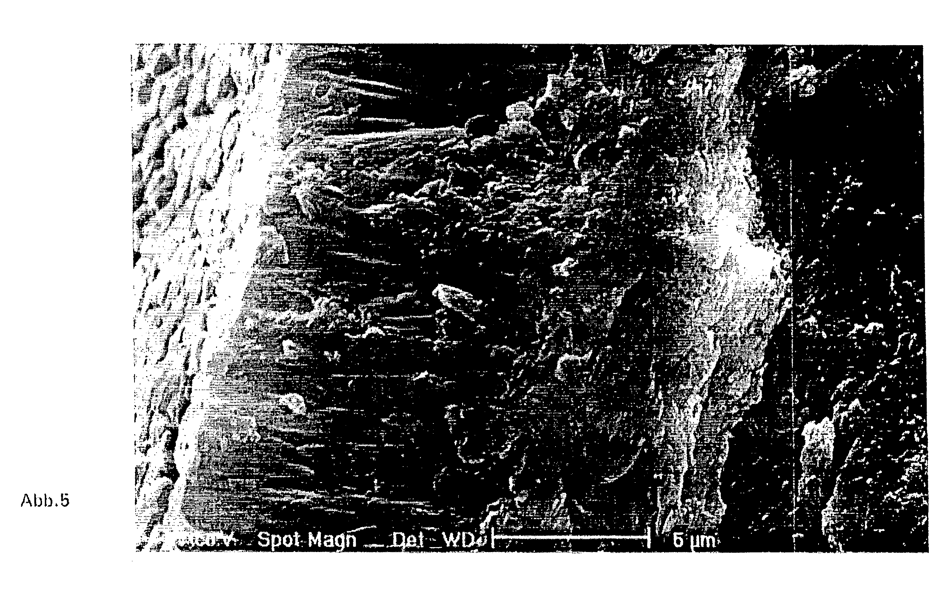

Das Vorgehen entspricht dem Beispiel 4a, die Schnittrichtung erfolgte jedoch parallel zur Längsachse der Schmelzprismen und die Probe wurde erst nach 10 Gel-Tauschzyklen untersucht. Die Schicht ist mit 9 µm fast doppelt so dick, wie die aus Beispiel 4 (vgl. Abbildung 5), was auf die Linearität der Wachstumsgeschwindigkeit hindeutet. Sie wirkt ausgereifter als die Schicht in Beispiel 4. Deutlich ist der fehlende Randspalt zwischen nativem und aufgewachsenem Material. Abbildung 4b zeigt die Aufsicht dieser Schicht, die morphologisch weitgehend repräsentativ für alle Schichten ist.

### Beispiel 6

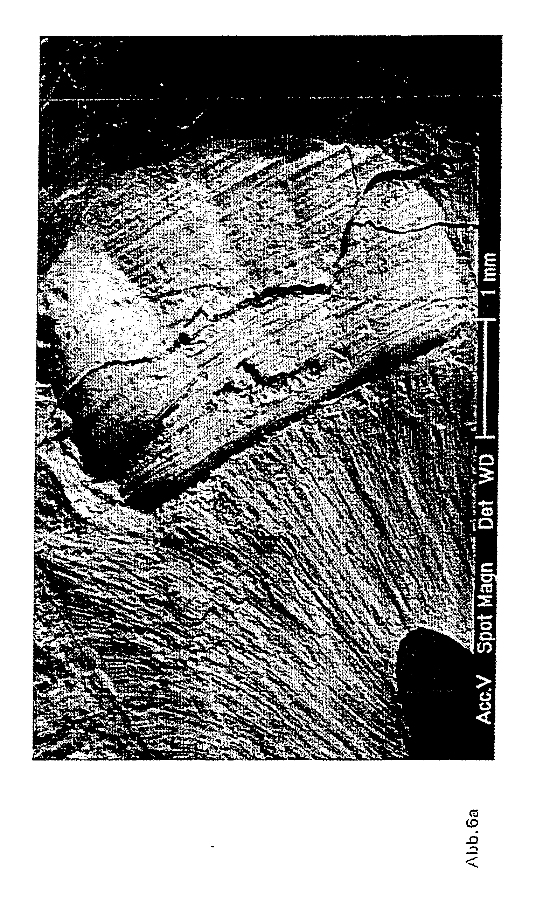

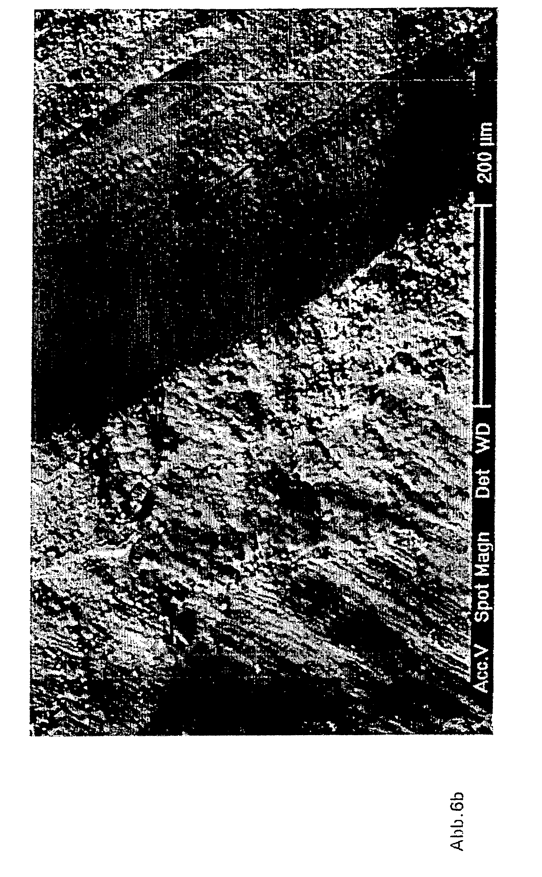

Um die Synthesebedingungen noch mehr der realen Problematik bei kariesbedingter Zahnhartsubstanzzerstörung anzupassen, wurde in die Kaufläche eines Prämolaren eine Kavität mit den Ausmaßen (H x B x T) 3 x 3 x 2 mm gebohrt. Die weitere Behandlung entspricht dem Beispiel 3. Die Calciumlösung enthielt jedoch 0,233 mol/l. Die Zahnprobe wurde dann zur Untersuchung so aufgebrochen, dass der Umriss der Kavität zu erkennen ist. Abbildungen 6a + b zeigen deutlich, dass sowohl der Kavitätsboden als auch die Seitenwände bewachsen sind.

Alle angeführten Synthesemethoden führen zu fest haftenden Fluorapatitschichten, die aufgrund der Kristallmorphologie und Anordnung große Ähnlichkeit zu nativem Zahnschmelz aufweisen. Die Orientierung der aufwachsenden Kristallite verläuft grundsätzlich senkrecht zur Oberfläche des Substrates. Durch entsprechend häufige Wiederholung der Austauschzyklen lassen sich prinzipiell beliebig dicke Schichten erzeugen.

## Patentansprüche

1. Verfahren zum Aufbau von Apatit auf Zahnmaterial umfassend die Schritte
(i) Auftragen eines ersten Gels, welches Gelatine sowie Phosphationen umfasst, auf einen Zahn,
(ii) Auftragen eines zweiten Gels, das frei ist von Phosphationen, Calciumionen und Fluoridionen, wobei mit diesem zweiten Gel die erste Gelschicht abgedeckt wird, und
(iii) Auftragen eines Calciumionen-enthaltenden Mediums.

2. Verfahren nach Anspruch 1,
**dadurch gekennzeichnet,**
**dass** als erstes Gel ein Gelatine-Glycerin-Gel eingesetzt wird.

3. Verfahren nach einem der vorhergehenden Ansprüche,
**dadurch gekennzeichnet,**
**dass** das erste Gel weiterhin Fluoridionen enthält.

4. Verfahren nach einem der vorhergehenden Ansprüche,
**dadurch gekennzeichnet,**
**dass** das erste Gel einen pH-Wert von 2,0 bis 6,0 aufweist.

5. Verfahren nach einem der vorhergehenden Ansprüche,
**dadurch gekennzeichnet,**
**dass** das zweite Gel ausgewählt wird aus Gelatine-Glyercin-Gelen, Polysaccharid-Gelen oder Carboxymethylcellulose-Gelen.

6. Verfahren nach einem der vorhergehenden Ansprüche,
**dadurch gekennzeichnet,**
**dass** man als Calciumionen enthaltendes Medium eine Calciumionen enthaltende Lösung oder eine Calciumionen enthaltendes Gel einsetzt.

7. Verfahren nach einem der vorhergehenden Ansprüche,
**dadurch gekennzeichnet,**
**dass** das Calciumionen-enthaltende Medium einen pH-Wert von 6 bis 8 aufweist.

8. Verfahren nach einem der vorhergehenden Ansprüche,
**dadurch gekennzeichnet,**
**dass** man das Zahnmaterial vor dem Auftragen des ersten Gels entfettet oder/und spült.

9. Verfahren nach einem der vorhergehenden Ansprüche,
**dadurch gekennzeichnet,**
**dass** es sich bei dem Zahnmaterial um Humanzähne oder/und Zahnschmelz handelt.

10. Verfahren nach einem der vorhergehenden Ansprüche,
**dadurch gekennzeichnet,**
**dass** die Komponenten (i), (ii) und (iii) zur Behandlung von kariösen Defekten durch Remineralisation vorgesehen sind.

11. Kit zur Verwendung im Verfahren gemäß den Ansprüchen 1 - 10, umfassend
(a) ein erstes Gel, welches Gelatine sowie Phosphationen umfasst,
(b) ein zweites Gel, welches frei von Phosphationen, Calciumionen und Fluoridionen ist sowie
(c) ein Calciumionen-enthaltendes Medium.

12. Kit nach Anspruch 11,
**dadurch gekennzeichnet,**
**dass** die Komponenten (a), (b) oder/und (c) weiterhin wie in einem der Ansprüche 2 bis 7 definiert sind.

## Claims

1. A process for building up apatite on tooth material, comprising the steps of
(i) applying a first gel, which comprises gelatin and phosphate ions, to a tooth,
(ii) applying a second gel which is free of phosphate ions, calcium ions and fluoride ions, wherein the first gel layer is covered with this second gel, and
(iii) applying a medium containing calcium ions.

2. A process according to Claim 1,
**characterised in that** a gelatin-glycerol gel is used as the first gel.

3. A process according to any one of the preceding Claims,
**characterised in that** the first gel further contains fluoride ions.

4. A process according to any one of the preceding Claims,
**characterised in that** the first gel has a pH value of 2.0 to 6.0.

5. A process according to any one of the preceding Claims,
**characterised in that** the second gel is selected from gelatin-glycerol gels, polysaccharide gels or carboxymethyl-cellulose gels.

6. A process according to any one of the preceding Claims,
**characterised in that** the medium containing calcium ions used is a solution containing calcium ions or a gel containing calcium ions.

7. A process according to any one of the preceding Claims,
**characterised in that** the medium containing calcium ions has a pH value of 6 to 8.

8. A process according to any one of the preceding Claims,
**characterised in that** the tooth material is defatted and/or rinsed before the application of the first gel.

9. A process according to any one of the preceding Claims,
**characterised in that** the tooth material is human teeth and/or dental enamel.

10. A process according to any one of the preceding Claims,
**characterised in that** the components (i),(ii) and (iii) are intended for the treatment of carious defects by remineralisation.

11. A kit for use in the process according to Claims 1 to 10, comprising
(a) a first gel which comprises gelatin and phosphate ions,
(b) a second gel which is free of phosphate ions, calcium ions and fluoride ions, and
(c) a medium containing calcium ions.

12. A kit according to Claim 11,
**characterised in that** the components (a),(b) and/or (c) are further defined as in any one of Claims 2 to 7.

## Revendications

1. Procédé pour la constitution d'apatite sur le matériau dentaire, comprenant les étapes consistant à :
(i) appliquer un premier gel qui comprend de la gélatine et des ions phosphate, sur une dent,
(ii) appliquer un deuxième gel qui est dépourvu d'ions phosphate, d'ions calcium et d'ions fluorure, la première couche de gel étant recouverte par ce deuxième gel, et
(iii) appliquer un milieu contenant des ions calcium.

2. Procédé selon la revendication 1,
**caractérisé en ce**
**que** l'on utilise comme premier gel, un gel de gélatine - glycérine.

3. Procédé selon l'une des revendications précédentes,
**caractérisé en ce**
**que** le premier gel contient des ions fluorure.

4. Procédé selon l'une des revendications précédentes,
**caractérisé en ce**
**que** le premier gel présente un pH de 2,0 à 6,0.

5. Procédé selon l'une des revendications précédentes,
**caractérisé en ce**
**que** le deuxième gel est choisi parmi les gels de gélatine - glycérine, les gels de polysaccharide ou les gels de carboxyméthylcellulose.

6. Procédé selon l'une des revendications précédentes,
**caractérisé en ce**
**que** l'on utilise comme milieu contenant des ions calcium, une solution contenant des ions calcium ou un gel contenant des ions calcium.

7. Procédé selon l'une des revendications précédentes,
**caractérisé en ce**
**que** le milieu contenant des ions calcium présente un pH de 6 à 8.

8. Procédé selon l'une des revendications précédentes,
**caractérisé en ce**
**que** l'on dégraisse et/ou l'on rince le matériau dentaire avant l'application du premier gel.

9. Procédé selon l'une des revendications précédentes,
**caractérisé en ce**
**que** le matériau dentaire est des dents humaines et/ou de l'émail dentaire.

10. Procédé selon l'une des revendications précédentes,
**caractérisé en ce**
**que** les composants (i), (ii) et (iii) sont prévus pour le traitement de défauts carieux par reminéralisation.

11. Kit à utiliser dans le procédé selon les revendications 1 à 10, comprenant
(a) un premier gel qui comprend de la gélatine et des ions phosphate,
(b) un deuxième gel qui est dépourvu d'ions phosphate, d'ions calcium et d'ions fluorure et
(c) un milieu contenant des ions calcium.

12. Kit selon la revendication 11,
**caractérisé en ce**
**que** les composants (a), (b) et/ou (c) sont tels que définis dans l'une des revendications 2 à 7.
